# EUROPÄISCHE PATENTSCHRIFT

(11) **EP 1 980 854 B1**
(45) Veröffentlichungstag und Bekanntmachung des Hinweises auf die Patenterteilung: **07.10.2009**
(21) Anmeldenummer: 07007510.6
(22) Anmeldetag: 12.04.2007
(51) Int. Cl.: G01N 33/543

(54) **Verfahren und Vorrichtung zur Messung der Konzentration eines in einer zu untersuchenden Probe enthaltenen Liganden**
Method and device for determining the ligand concentration in a sample under investigation
Procédé et dispositif destinés à la mesure de la concentration de ligands contenus dans un échantillon à analyser

(43) Veröffentlichungstag der Anmeldung: 15.10.2008
(73) Patentinhaber: Micronas Holding GmbH, 79108 Freiburg (DE)
(72) Erfinder: Sieben, Ulrich, Dr. rer. nat., 79279 Vörstetten (DE); Klapproth, Holger, Dr., 79108 Freiburg (DE)
(74) Vertreter: Huwer, Andreas

(56) Entgegenhaltungen:
- WO-A-20/04031750
- WO-A1-01/08799

## Beschreibung

Die Erfindung betrifft ein Verfahren zur Messung der Konzentration mindestens eines in einer zu untersuchenden Probe enthaltenen Liganden, wobei an mindestens zwei Teststellen, die an der Oberfläche mindestens eines Trägers angeordnet sind, jeweils mindestens ein Rezeptor immobilisiert wird, der dazu geeignet ist, beim Kontaktieren des Liganden spezifisch an diesen zu binden. Außerdem bezieht sich die Erfindung auf eine Vorrichtung zur Messung der Konzentration mindestens eines in einer zu untersuchenden Probe enthaltenen Liganden, mit einer zumindest eine Einlassöffnung für die Probe aufweisenden Messkammer, die eine Innenhöhlung mit Begrenzungswänden aufweist, wobei an wenigstens einer Begrenzungswand mindestens zwei Teststellen angeordnet sind, an denen jeweils zumindest ein Rezeptor immobilisiert ist, der dazu geeignet ist, beim Kontaktieren des Liganden spezifisch an diesen zu binden, und mit mindestens einem Sensor zur Erfassung von Messsignalen für die Anzahl oder die Dichte der Bindungsereignisse an den einzelnen Teststellen.

Ein derartiges Verfahren und eine derartige Vorrichtung sind aus DE 102 45 435 B4 bekannt. Die Vorrichtung hat eine als Flusszelle ausgebildete Messkammer mit einer eine Einlass- und einer Auslassöffnung für die zu untersuchende Probe aufweisenden Innenhöhlung. Die Innenhöhlung ist von Begrenzungswänden umgrenzt, nämlich einem planen Bodenteil, einem parallel dazu beabstandeten planen Deckenteil und seitlichen Begrenzungswänden, an denen die Ein- und Auslassöffnungen vorgesehen sind. Das Bodenteil hat an seiner an die Innenhöhlung angrenzenden Oberfläche eine optische Wellenleiterschicht, auf der mehrere seitlich voneinander beabstandete Teststellen angeordnet sind, an denen Rezeptoren im Evaneszenzfeld des Wellenleiters immobilisiert sind. Zur Anregung der Emission von Lumineszenzstrahlung in Abhängigkeit von der Bindung des in der Probe enthaltenen Liganden an die Rezeptoren wird in den Wellenleiter eine optische Strahlung eingekoppelt, die mit Hilfe einer Strahlungsquelle erzeugt wird. Unter den Rezeptoren ist an jeder Teststelle jeweils ein Strahlungsempfänger vorgesehen, der für die Lumineszenzstrahlung empfindlich ist. Obwohl sich die Vorrichtung wegen ihres einfachen und kostengünstigen Aufbaus bewährt hat, weist sie dennoch Nachteile auf So ist die Messung der Konzentration des Liganden auf einen vorgegebenen Messbereich begrenzt, insbesondere wenn an den einzelnen Teststellen für mehrere unterschiedliche Liganden gleichzeitig die Konzentration gemessen werden soll. Liegt die Konzentration mindestens eines Liganden außerhalb des Messbereichs, kann es vorkommen, dass die Messwerte für die betreffende Teststelle in Begrenzung geraten.

Es besteht deshalb die Aufgaben ein Verfahren und eine Vorrichtung der eingangs genannten Art zu schaffen, die über einen breiten Konzentrationsbereich mit großer Präzision eine Messung der Konzentration des Liganden ermöglicht.

Diese Aufgabe wird bezüglich des Verfahrens der eingangs genannten Art dadurch gelöst, dass die Probe für eine vorbestimmte Zeitdauer derart mit den Teststellen in Kontakt gebracht wird, dass in der Probe an die an den Teststellen immobilisierten Rezeptoren unterschiedlich große Diffusionsvolumina angrenzen, aus denen der mindestens eine Ligand während der vorbestimmten Zeitdauer jeweils zu dem betreffenden Rezeptor diffundieren kann, wenn der Ligand in dem Diffusionsvolumen enthalten ist, und dass nach Ablauf der vorbestimmten Zeitdauer für jede Teststelle jeweils ein Messsignal für die Anzahl oder die Dichte der Bindungsereignisse an der Teststelle erfasst wird.

In vorteilhafter Weise ergeben sich durch die unterschiedlich großen und ggf unterschiedliche Abmessungen aufweisenden Diffusionsräume bei der Messung für die einzelnen Teststellen unterschiedliche Kalibrationskurven oder Kennlinien (Messsignal als Funktion der Expositionsdauer für eine bestimmte Ligandenkonzentration). Wenn zu Beginn eines Versuchs die Ligandenkonzentration an den einzelnen Teststellen jeweils die gleiche ist, der Ligand also homogen in der Probe verteilt ist, wird das Messsignal der Teststelle, die an ein großes Diffusionsvolumen angrenzt, schneller in die Begrenzung geraten als das Messsignal einer Teststelle, die an ein kleineres Diffusionsvolumen angrenzt, weil aus dem größeren Diffusionsvolumen pro Zeiteinheit mehr Liganden zu den Rezeptoren an der betreffenden Teststelle diffundieren können als bei dem kleinen Diffusionsvolumen. Dadurch wird ermöglicht, dass sich über einen weiten Konzentrationsbereich jeweils mindestens ein Messsignal in einem günstigen Aussteuerungsbereich befindet. Der mindestens eine Rezeptor kann eine Nukleinsäure oder ein Derivate davon (DNA, RNA, PNA, LNA, Oligonukleotide, Plasmide, Chromosomen), ein Peptid, ein Protein (Enzym, Protein, Oligopeptide, zelluläres Rezeptorprotein und dessen Komplexe, Peptidhormon, Antikörper und dessen Fragmente), ein Kohlenhydrat und dessen Derivate, insbesondere ein glykosyliertes Protein und ein Glycosid, ein Fett, eine Fettsäure und/oder ein Lipid umfassen.

Bei einer bevorzugten Ausführungsform der Erfindung werden die Messwerte jeweils mit einem vorgegebenen Referenzwert oder Referenzbereich verglichen und mit Hilfe des Messsignals, das den geringsten Abstand zum Referenzwert oder Referenzwertbereich aufweist oder mit diesem übereinstimmt, wird die Konzentration des Liganden in der Probe bestimmt. Durch den Vergleich der einzelnen Messergebnisse mit dem Referenzwert oder Referenzbereich kann ein Messsignal ausgewählt werden, das sich nach Ablauf der Mess- bzw. Expositionsdauer in einem günstigen Aussteuerungsbereich befindet und daher eine präzise Bestimmung der Ligandenkonzentration ermöglicht. Dabei ist es sogar möglich, dass die Konzentration mit Hilfe von mehreren Messsignalen, die bei Ablauf der Mess- bzw. Expositionsdauer innerhalb des Referenzbereichs liegen, ermittelt wird, indem beispielsweise ein Mütelwert gebildet wird.

Vorteilhaft ist, wenn der Referenzwert zwischen 25% und 75%, insbesondere zwischen 40% und 60% des Messsignals liegt, das an einer Teststelle gemessen wird, wenn im Wesentlichen alle Rezeptoren der Teststelle an einen Ligand gebunden sind, und wenn der Referenzwert bevorzugt dem Wendepunkt einer sigmoidalen Kalibrationskurve entspricht, die unterschiedlichen Werten für die Ligandenkonzentration jeweils einen Messwert für die Teststelle zuordnet. Das Verfahren ermöglicht dann einen noch genauere Konzentrationsmessung, insbesondere wenn bei der Messung Messwerte für eine entsprechend große Anzahl von Teststellen mit unterschiedlichen Diffusionsräumen erfasst werden.

Bei einer vorteilhaften Ausgestaltung des Verfahrens werden die unterschiedlich großen Diffusionsvolumina durch mindestens eine Begrenzungswand gebildet, die mit Abstand zu der Teststelle an die Probe angrenzt. Das Diffiusionsvolumen ist dann durch die Geometrie der Messkammer vorgegeben und somit leicht reproduzierbar.

Bei einer bevorzugten Ausführungsform der Erfindung werden die unterschiedlich großen Diffusionsvolumina dadurch gebildet, dass die Probe mit einer unterschiedlichen Füllstandshöhe an den einzelnen Teststellen angeordnet wird. Dies kann beispielsweise dadurch erreicht werden, dass Teststellen am Boden eines Trogs oder einer Messkammer vorgesehen sind und dass der Boden gegenüber einer Horizontalebene schräg angeordnet ist, so dass die Füllstandshöhe der Probe ausgehend vom einen Randbereich des Bodens zum diametral gegenüberliegenden anderen Randbereich des Bodens beispielsweise keilförmig zu oder abnimmt.

Bezüglich der Vorrichtung der eingangs genannten Art wird die vorstehend genannte Aufgabe dadurch gelöst, dass bei den einzelnen Teststellen der Abstand zwischen der Teststelle und einem dieser jeweils in Richtung der Normalen auf die Erstreckungsebene der Teststelle gegenüberliegenden Wandungsbereich der Messkammer unterschiedlich groß ist.

Wenn die Messkammer mit der Probe befüllt ist, ergeben sich dann an den einzelnen Teststellen unterschiedlich große Diffusionsvolumina in der Probe, aus denen während einer vorgegebenen Messdauer Liganden zu den an der Teststelle befindlichen Rezeptoren diffundieren können, um spezifisch an diese zu binden. Somit sind die Messsignale an den einzelnen Teststellen nach Beendigung der Messdauer unterschiedlich ausgesteuert. Dadurch wird ermöglicht, dass sich über einen weiten Konzentrationsbereich jeweils mindestens ein Messsignal in einem günstigen Aussteuerungsbereich befindet, in dem die Konzentration des Liganden mit großer Präzision bestimmt werden kann.

Vorteilhaft ist, wenn der mindestens eine Sensor mit einer Auswerteeinrichtung verbunden ist, wenn die Auswerteeinrichtung einen Referenzwertgeber zum Bereitstellen mindestens eines Referenzwerts oder Referenzbereichs für die Messsignale der Teststellen aufweist, wenn die Auswerteeinrichtung mindestens eine Vergleichseinrichtung zum Vergleichen der Messsignale der einzelnen Teststellen mit dem Referenzwert oder Referenzwertbereich hat, und wenn die Vergleichseinrichtung derart mit einer Schalteinrichtung verbunden ist, dass das Messsignal, welches den geringsten Abstand zum Referenzwert oder Referenzwertbereich aufweist oder mit diesem übereinstimmt, an einen Messsignalausgang der Schalteinrichtung anlegbar ist. Mit Hilfe der Auswerteeinrichtung kann dann durch Vergleich mit dem Referenzwert oder Referenzwertbereich ein für die Messung der jeweiligen Ligandenkonzentration günstig ausgesteuertes Messsignal ausgewählt und an den Messsignalausgang angelegt werden. Mit Hilfe dieses Messsignals und der entsprechenden Teststelle zugeordneten Kenngrößen, beispielsweise einer Kalibrationskurve, kann dann die Konzentration des Liganden in der Probe mit große Präzision bestimmt werden. Die Auswerteeinrichtung weist bevorzugt einen Mikrocomputer auf Der Messsignalausgang kann in dem Mikrocomputer angeordnet sein, insbesondere in Form eines Ausgangs für ein serielles oder paralleles Digitalsignal.

Eine besonders genaue Messung der Konzentration des Liganden wird dadurch ermöglicht, dass der Referenzwert dem Wendepunkt einer sigmoidalen Kalibrationskurve entspricht, die unterschiedlichen Werten für die Ligandenkonzentration jeweils einen Messwert für die Teststelle zuordnet.

Vorteilhaft ist, wenn mindestens eine Teststelle an einer ersten Kammerwand und der dieser Teststelle gegenüberliegende Wandungsbereich an einer zweiten Kammerwand angeordnet sind, und wenn diese Kammerwände unter einem Winkel relativ zueinander geneigt sind. Dabei ist es sogar möglich, dass die Kammerwände in quer zueinander verlaufenden Richtungen relativ zueinander geneigt sind. Die Vorrichtung ist dann kostengünstig mit einer Vielzahl unterschiedlicher Diffusionsvolumina herstellbar.

Der Neigungswinkel kann mindestens 2,5°, insbesondere mindestens 5° und vorzugsweise mindestens 10° betragen. Dabei wird unter einem Neigungswinkel der Winkel verstanden, unter dem die Haupterstreckungsebenen der einander gegenüberliegenden, vorzugsweise ebenen Kammerwände relativ zueinander verkippt sind. Die Teststellen sind bevorzugt an mindestens einer der beiden relativ zueinander geneigten Kammerwände angeordnet.

Bei einer anderen vorteilhaften Ausgestaltung der Erfindung ist zwischen mindestens zwei Wandungsbereichen eine Stufe oder ein Absatz gebildet, wobei jedem dieser Wandungsbereiche jeweils mindestens eine Teststelle zugeordnet ist. Eine Messkammer mit einer solchen, gestuften Innenwand ist mit Methoden der Halbleiterfertigungstechnik kostengünstig mit großer Präzision serienmäßig herstellbar.

Gegebenenfalls ist es sogar möglich, dass die erste Kammerwand und/oder die dieser gegenüberliegende zweite Kammerwand in quer zueinander verlaufenden Richtungen jeweils mehrere nebeneinander angeordnete Wandungsbereiche aufweisen, zwischen denen Stufen oder Absätze gebildet sind. Die Vorrichtung ermöglicht dann bei kompakten Abmessungen eine Vielzahl von Teststellen, die an unterschiedliche Diffusionsvoluming angrenzen.

Kompakte Abmessungen der Messkammer werden auch dadurch ermöglicht, dass die Wandungsbereiche, zwischen denen die Stufen oder Absätze gebildet sind, als Vielecke ausgestaltet und vorzugsweise schachbrettmusterartig oder wabenartig angeordnet sind. Die Messkammer lässt sich dann mit Standardprozessen der Halbleiterfertigung kostengünstig herstellen.

Vorteilhaft ist, wenn mindestens eine erste Teststelle in Richtung der Normalen auf die von ihr aufgespannte Ebene einen ersten Abstand zum gegenüberliegenden Wandungsbereich und wenigstens eine zweite Teststelle in Richtung der Normalen auf die von ihr aufgespannte Ebene einen zweiten Abstand zum gegenüberliegenden Wandungsbereich aufweist, und wenn der erste Abstand mindestens das 1,5-fache, insbesondere mindestens das zweifache und vorzugsweise mindestens das vierfache des zweiten Abstands beträgt. Mit diesen Abmessungen wird eine hohe Messdynamik der Vorrichtung ermöglicht.

Zweckmäßigerweise ist an den einzelnen Teststellen jeweils mindestens ein Sensor zum Detektieren der Liganden-Rezeptor-Komplexe in der Wandung der Messkammer angeordnet, wobei der wenigstens eine Rezeptor auf dem Sensor immobilisiert ist. Die Liganden-Rezeptor-Komplexe können dann direkt vor Ort und somit mit entsprechend großer Nachweisempfindlichkeit detektiert werden.

Der mindestens eine Sensor kann ein ionenselektiver Feldeffekttransistor sein. Die Vorrichtung ist dann kostengünstig herstellbar.

Bei einer bevorzugten Ausgestaltung der Erfindung ist mindestens ein Sensor ein optischer Strahlungsempfänger, der für eine in Abhängigkeit von der Bindung des Liganden an den Rezeptor anregbare Lumineszenzstrahlung empfindlich ist. Die Lumineszenzstrahlung kann durch eine Anregungsstrahlung angeregt werden, die mittels einer Strahlungsquelle erzeugt werden kann. Die Strahlungsquelle kann als Halbleiterbauelement ausgebildet sein, das in die Wandung der Messkammer integriert ist. Die Lumineszenzstrahlung kann aber auch chemisch angeregt werden. Dadurch kann eine Strahlungsquelle eingespart werden.

Nachfolgend sind Ausführungsbeispiele der Erfindung anhand der Zeichnung näher erläutert. Es zeigt:
- Fig. 1: einen Querschnitt durch ein erstes Ausführungsbeispiel einer Vorrichtung zur Messung der Konzentration eines in einer zu untersuchenden Probe enthaltenen Liganden,
- Fig. 2: eine vergrößerte Teilansicht von Fig. 1, die eine Teststelle zeigt, an der Rezeptoren auf einem optischen Sensor immobilisiert sind,
- Fig. 3: eine schematische Darstellung einer Auswerteeinrichtung der Vorrichtung,
- Fig. 4: eine graphische Darstellung einer Kalibrationskurve für ein Testfeld der Vorrichtung, wobei auf der Abszisse die Zeit und auf der Ordinate die Amplitude eines Messsignals für die Teststelle aufgetragen ist,
- Fig. 5: einen Querschnitt durch ein zweites Ausführungsbeispiel der Vorrichtung,
- Fig. 6: einen Querschnitt durch ein drittes Ausführungsbeispiel der Vorrichtung, und
- Fig. 7: einen Längsschnitt durch das dritte Ausführungsbeispiel.

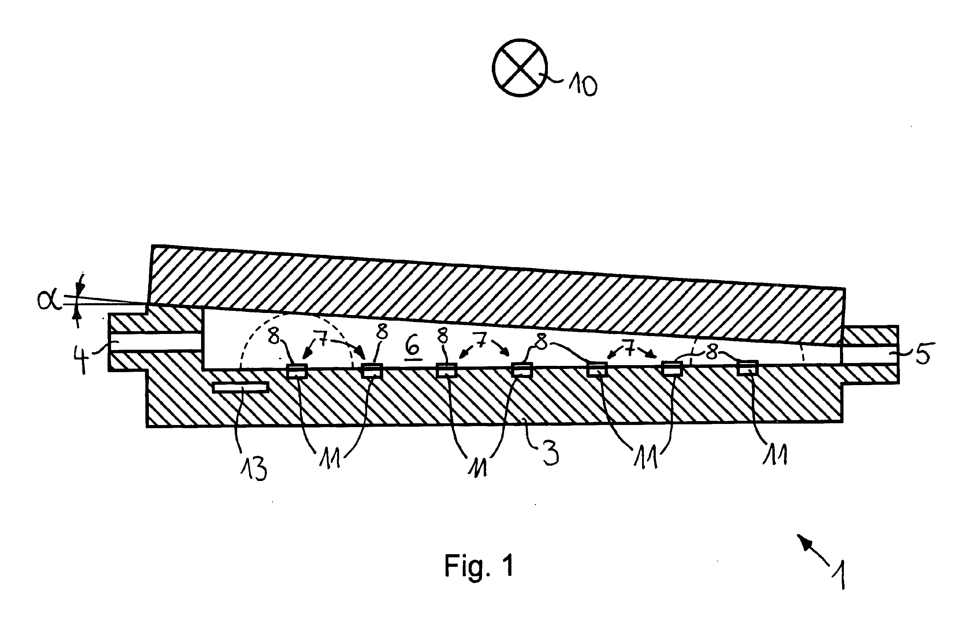

Eine in Fig. 1 im Ganzen mit 1 bezeichnete Vorrichtung zur Messung der Konzentration mehrerer eines in einer zu untersuchenden Probe enthaltener Liganden 2 unterschiedlicher Ligandenarten hat eine Messkammer 3 mit einer bis auf eine Einlassöffnung 4 und eine Auslassöffnung 5 geschlossenen Innenhöhlung 6. Die Innenhöhlung 6 ist nach oben, unten und seitlich durch Begrenzungswände begrenzt.

An einer den Boden der Messkammer 3 bildenden Begrenzungswand sind mehrere voneinander beabstandete Teststellen 7 vorgesehen, die matrixförmig in Reihen und Spalten nebeneinander angeordnet sind. An den Teststellen 7 der einzelnen Reihen ist jeweils ein Rezeptor 8 immobilisiert, der für einen Ligand 2 einer bestimmten Ligandenart bindungsspezifisch ist. Die Rezeptoren einer ersten Teststellenreihe unterscheiden sich von den Rezeptoren einer zweiten Teststellenreihe. Selbstverständlich ist es auch möglich, dass die Rezeptoren 8 mehrerer Reihen mit Teststellen 7 oder aller Teststellen 7 derselben Rezeptorart angehören.

Wenn in der Messkammer 3 Rezeptoren 8 für mindestens zwei Rezeptorarten immobilisiert sind, müssen diese nicht notwendigerweise in derselben Reihe der Teststellen-Matrix angeordnet sein. Vielmehr können in einer Teststellen-Reihe auch Rezeptoren unterschiedlicher Rezeptorarten angeordnet sein, wobei jedoch an ein und derselben Teststelle 7 immer nur Rezeptoren derselben Rezeptorart immobilisiert sind. An eine bestimmte Teststelle 7 können also jeweils nur Liganden einer vorbestimmten Ligandenart binden.

An den einzelnen Teststellen 7 ist der Abstand zwischen der Teststelle 7 und einem dieser jeweils in Richtung der Normalen auf die Erstreckungsebene der Teststelle 7 gegenüberliegenden Wandungsbereich der Messkammer unterschiedlich groß. Wenn die Innenhöhlung 6 mit der Probe befüllt ist, grenzen dadurch in der Probe unterschiedlich große Diffusionsvolumina, aus denen der mindestens eine Ligand während einer vorbestimmten Expositionsdauer jeweils zu den an der betreffenden Teststelle 7 befindlichen Rezeptoren 8 diffundieren kann, an die Teststelle 7 an.

Unter einem Diffusionsvolumen wird das innerhalb einer Kugel, deren Mittelpunkt an der Teststelle liegt und deren Radius der mittleren Diffusionsgeschwindigkeit des Liganden in der Probe multipliziert mit der Expositionsdauer entspricht, liegende Teilvolumen der Innenhöhlung 6 der Messkammer 3 verstanden. Unter einer Expositionsdauer wird die Zeitdauer verstanden, während der die Probe bis zum Beenden der Messung mit der Teststelle 7 in Kontakt steht. In Fig. 1 ist das Diffusionsvolumen für zwei der Teststellen 7 strichliniert angedeutet. Deutlich ist erkennbar, dass der links im Bild befindlichen Teststelle 7 ein größeres Diffusionsvolumen zugeordnet ist als der rechten Teststelle 7.

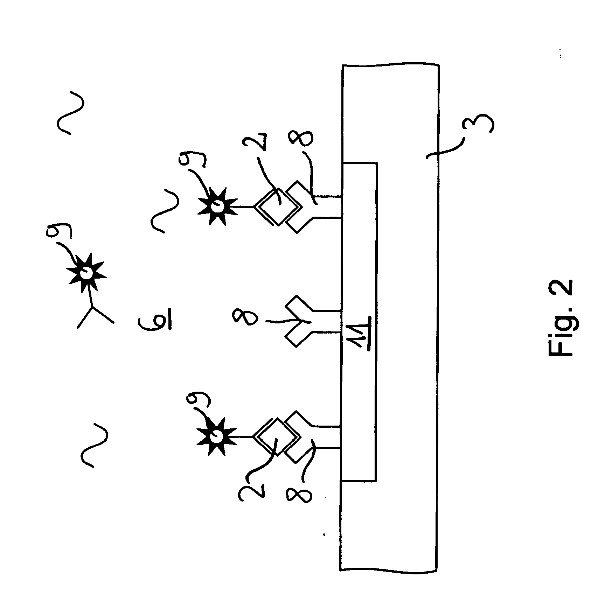

In Fig. 2 ist erkennbar, dass die Liganden 2 über einen Nachweisantikörper mit einem optischen Marker 9 markiert sind. Bevorzugt wird zunächst die Probe für die vorbestimmte Expositionsdauer mit den Rezeptoren 8 in Kontakt gebracht und danach werden nicht an einen Rezeptor 8 gebundene Bestandteile der Probe aus der Messkammer 3 enifernt, beispielsweise indem eine Spülflüssigkeit durch die Messkammer 3 geleitet wird. Danach werden die an einen Rezeptor 8 gebundenen Liganden 2 mit dem Marker 9 markiert, indem die Innenhöhlung 6 der Messkammer 3 mit einer Flüssigkeit befüllt wird, die den Marker 9 enthält. Nachdem die Marker 9 an die Liganden 2 gebunden haben, werden freie Marker 9 aus der Messkammer 3 entfernt, beispielsweise durch erneutes Spülen der Innenhöhlung 6.

Es ist aber auch möglich, dass die Liganden 2 mit dem Marker 9 markiert werden, bevor die Probe mit den Teststellen 7 in Kontakt gebracht wird. In diesem Fall wird die Probe mit den darin enthaltenen, markierten Liganden 2 zunächst für eine vorbestimmte Expositionsdauer mit den Teststellen 7 in Kontakt gebracht und danach werden nicht an einen Rezeptor 8 gebundene Bestandteile der Probe aus der Messkammer 3 entfernt.

Nachdem die Liganden 2 an die Rezeptoren 8 gebunden und markiert wurden, werden die Teststellen mit Hilfe einer Strahlungsquelle 10 mit einer optischen Anregungsstrahlung bestrahlt, welche die Marker 9 zur Aussendung von Lumineszenzstrahlung anregt. Die Wellenlänge der Lumineszenzstrahlung unterscheidet sich von der Wellenlänge der Anregungsstrahlung.

Die Lumineszenzstrahlung wird mit Hilfe von optischen Sensoren 11 detektiert, die an den einzelnen Teststellen 7 jeweils direkt unter den Rezeptoren 8 in die Wandung der Messkammer 3 integriert sind. Zwischen den Rezeptoren 8 und den Sensoren 11 ist ein in der Zeichnung nicht näher argestelltes optisches Sperrfilter für die Anregungsstrahlung vorgesehen, das für die Lumineszenzstrahlung durchlässig ist. Mit Hilfe der Sensoren 11 wird für jede Teststelle 7 jeweils ein Messsignal erzeugt, dessen Amplitude von der Anzahl der Marker an der betreffenden Teststelle 7 abhängig ist.

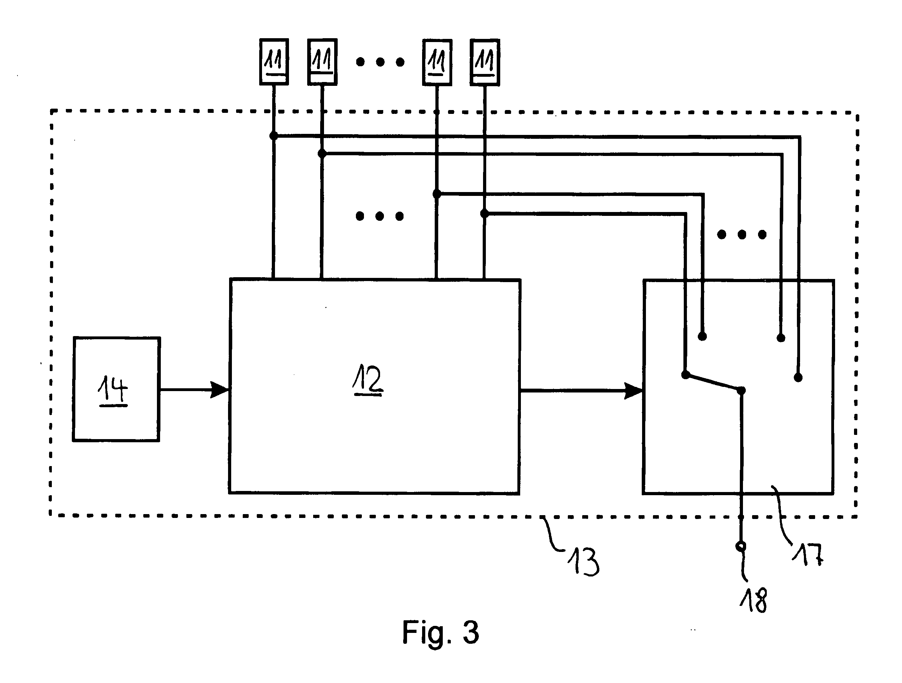

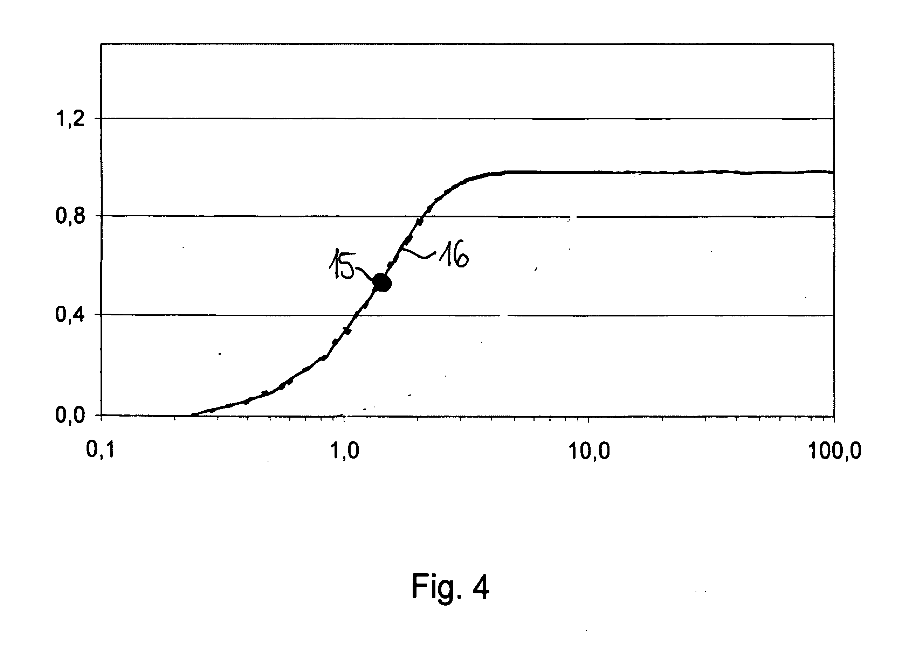

Wie in Fig. 3 erkennbar ist, haben die Sensoren 11 jeweils einen Messsignalausgang, der mit einem Eingang einer Vergleichseinrichtung 12 einer Auswerteeinrichtung 13 verbunden ist. Ein weiterer Eingang der Vergleichseinrichtung 12 ist an einem Referenzwertgeber 14 angeschlossen, der einen Referenzwert bereitstellt. Der Referenzwert entspricht dem Wendepunkt 16 einer sigmoidalen Kalibrationskurve 17, die unterschiedlichen Werten für die Ligandenkonzentration jeweils einen Messwert für die betreffende Teststelle 7 zuordnet (Fig. 4). Mit Hilfe der Vergleichseinrichtung 12 werden die Messsignale der einzelnen Sensoren 11 jeweils mit dem Referenzwert verglichen. Die Vergleichseinrichtung 12 steht derart mit einer Schalteinrichtung 17 in Steuerverbindung, dass das Messsignal, welches den geringsten Abstand zum Referenzwert aufweist oder mit diesem übereinstimmt, an einen Messsignalausgang der Schalteinrichtung angelegt wird. Mit Hilfe dieses Messsignals und einer Kalibrationskurve, wird die Konzentration des Liganden 2 in der Probe bestimmt. In der Kalibrationskurve sind Kenngrößen für das Diffusionsvolumen an der dem Messsignal zugeordneten Teststelle 7 und die Bindungskonstante der an der Teststelle 7 befindlichen Rezeptoren 8 berücksichtigt. Die Kalibrationskurve kann mit Hilfe mindestens eines Versuchs durch Messen ermittelt und/oder rechnerisch, beispielsweise mit Hilfe eines geeigneten Modells, erstellt werden.

In Fig. 3 ist die Auswerteeinrichtung 13 nur schematisch dargestellt. Sie kann insbesondere einen Mikrocomputer aufweisen, in dem die einzelnen Messsignale mittels eines in dem Mikrocomputer ablaufenden Betriebsprogramms mit dem Referenzwert verglichen werden. Der Referenzwertgeber kann einen Datenspeicher aufweisen, in dem der Referenzwert vorzugsweise in digitaler Form abgelegt ist. Die einzelnen Messsignale können nacheinander oder gleichzeitig mit dem Referenzwert verglichen werden. Es ist auch denkbar, dass der Referenzwertgeber 14 für jede Teststelle 7 und/oder für jede Rezeptorart einen eigenen Referenzwert bereitstellt.

Bei dem in Fig. 1 gezeigten Ausführungsbeispiel sind die der Innenhöhlung 6 zugewandten Innenflächen der Messkammer-Begrenzungswand, an der die Teststellen 7 vorgesehen sind, und die dieser gegenüberliegende Begrenzungswand unter einem Winkel α relativ zueinander geneigt. Die Innenflächen beider Begrenzungswände sind eben, so dass die Innenhöhlung 6 einen etwa keilförmigen Querschnitt aufweist.

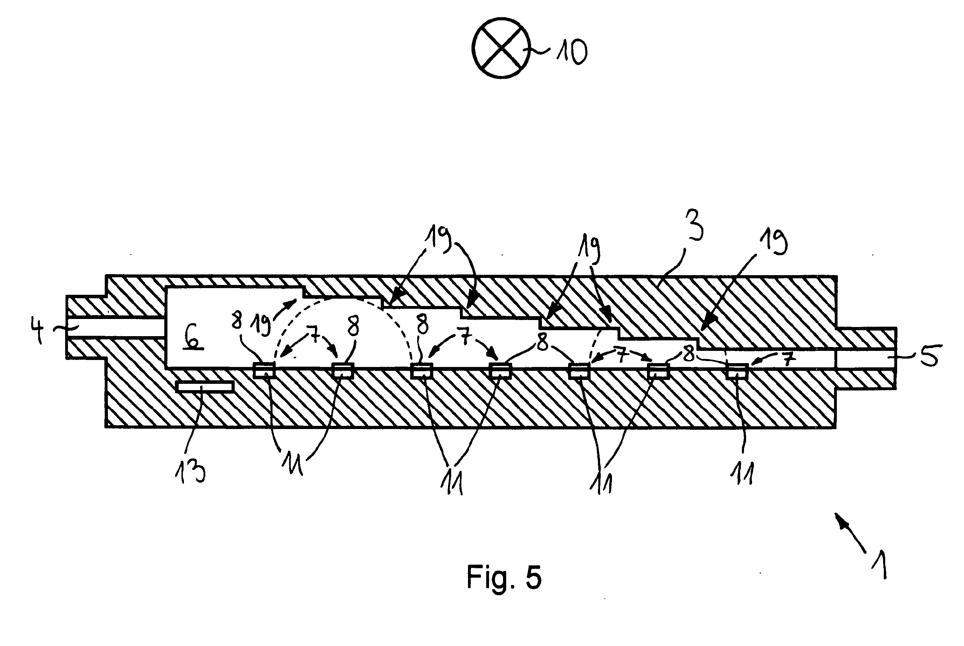

Bei den Ausführungsbeispielen gemäß Fig. 5 bis 7 weist die den Teststellen 7 gegenüberliegende Begrenzungswand, nämlich die des Deckelteils der Messkammer 3, an ihrer der Innenhöhlung 6 zugewandten Innenfläche Stufen 19 auf Jeder Stufe 19 liegt jeweils eine Teststelle 7 etwa mittig gegenüber. Der der Teststelle 7 zugewandte Wandungsbereich der Stufe 19 verläuft jeweils etwa parallel zur Innenfläche der gegenüberliegenden, die Teststelle 7 aufweisenden Begrenzungswand.

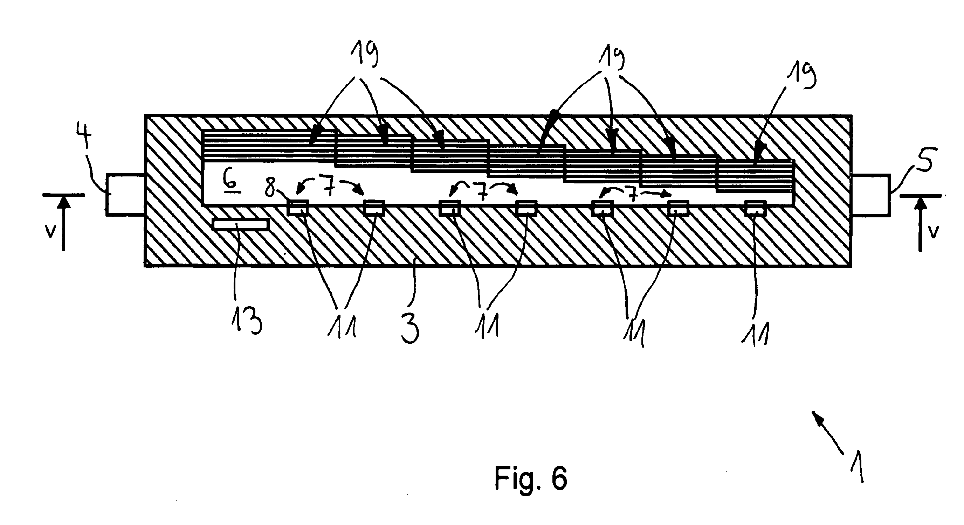

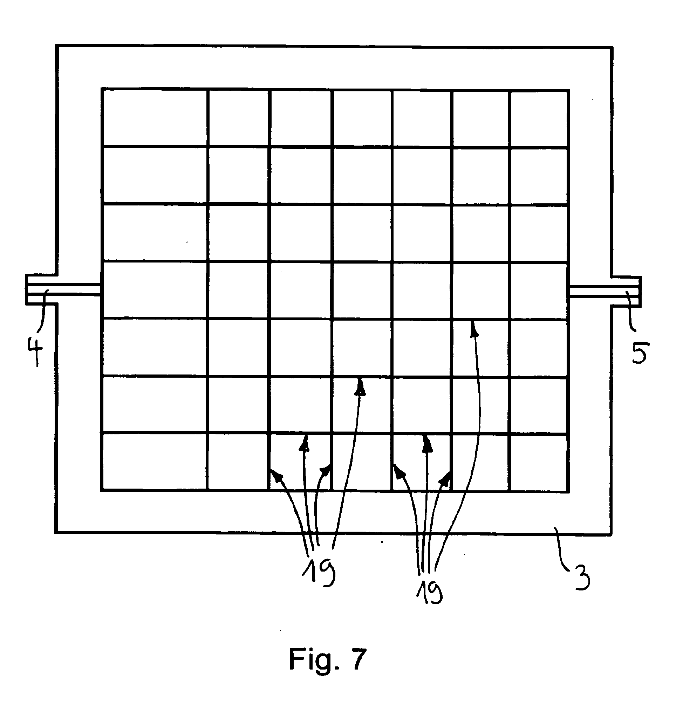

Bei dem in Fig. 6 und 7 gezeigten Ausführungsbeispiel ist die den Teststellen 7 gegenüberliegende Begrenzungswand der Innenhöhlung 6 in rechtwinklig zueinander verlaufenden Richtungen gestuft. In der Aufsicht auf die Begrenzungswand sind die einzelnen, den Teststellen 7 zugewandten, durch die Stufen gebildeten Wandungsbereiche der Messkammer 3 matrixförmig in mehreren Reihen und Spalten nebeneinander angeordnet. Dadurch ergibt sich ein etwa schachbrettartiges Stufenmuster.

Die Sensoren 11 zur Detektion der Bindungsereignisse sind als ionenselektive Feldeffekttransistoren ausgestaltet, die direkt unter den Rezeptoren 8 in die Wandung der Messkammer 3 integriert sind. Auch die Auswerteeinrichtung 13 ist in die Wandung der Messkammer 3 integriert.

## Patentansprüche

1. Verfahren zur Messung der Konzentration mindestens eines in einer zu untersuchenden Probe enthaltenen Liganden (2), wobei an mindestens zwei Teststellen (7), die an der Oberfläche mindestens eines Trägers angeordnet sind, jeweils mindestens ein Rezeptor (8) immobilisiert wird, der dazu geeignet ist, beim Kontaktieren des Liganden (2) spezifisch an diesen zu binden, wobei die Probe für eine vorbestimmte Zeitdauer derart mit den Teststellen (7) in Kontakt gebracht wird, dass in der Probe an die an den Teststellen (7) immobilisierten Rezeptoren (8) unterschiedlich große Diffusionsvolumina angrenzen, aus denen der mindestens eine Ligand (2) während der vorbestimmten Zeitdauer jeweils zu dem betreffenden Rezeptor (8) diffundieren kann, wenn der Ligand (2) in dem Diffusionsvolumen enthalten ist, wobei nach Ablauf der vorbestimmten Zeitdauer für jede Teststelle (7) jeweils ein Messsignal für die Anzahl oder die Dichte der Bindungsereignisse an der Teststelle (7) erfasst wird.

2. Verfahren nach Anspruch 1, **dadurch gekennzeichnet, dass** die Messwerte jeweils mit einem vorgegebenen Referenzwert oder Referenzbereich verglichen werden, und dass mit Hilfe des Messsignals, das den geringsten Abstand zum Referenzwert oder Referenzwertbereich aufweist oder mit diesem übereinstimmt, die Konzentration des Liganden (2) in der Probe bestimmt wird.

3. Verfahren nach Anspruch 1 oder 2, **dadurch gekennzeichnet, dass** der Referenzwertbereich zwischen 25% und 75%, insbesondere zwischen 40% und 60% des Messsignals liegt, das an einer Teststelle (7) gemessen wird, wenn im Wesentlichen alle Rezeptoren (8) der Teststelle (7) an einen Ligand (2) gebunden sind, und dass der Referenzwert bevorzugt dem Wendepunkt (15) einer sigmoidalen Kalibrationskurve (16) entspricht, die unterschiedlichen Werten für die Ligandenkonzentration jeweils einen Messwert für die Teststelle (7) zuordnet.

4. Verfahren nach einem der Ansprüche 1 bis 3, **dadurch gekennzeichnet, dass** die unterschiedlich großen Diffusionsvolumina durch mindestens eine Begrenzungswand gebildet werden, die mit Abstand zu der Teststelle an die Probe angrenzt.

5. Verfahren nach einem der Ansprüche 1 bis 4, **dadurch gekennzeichnet, dass** die unterschiedlich großen Difiusionsvolumina dadurch gebildet werden, dass die Probe mit einer unterschiedlichen Füllstandshöhe an den einzelnen Teststellen (7) angeordnet wird.

6. Vorrichtung (1) zur Messung der Konzentration mindestens eines in einer zu untersuchenden Probe enthaltenen Liganden, mit einer zumindest eine Einlassöffnung (4) für die Probe aufweisenden Messkammer (3), die eine Innenhöhlung (6) mit Begrenzungswänden aufweist, wobei an wenigstens einer Begrenzungswand mindestens zwei Teststellen (7) angeordnet sind, an denen jeweils zumindest ein Rezeptor (8) immobilisiert ist, der dazu geeignet ist, beim Kontaktieren des Liganden (2) spezifisch an diesen zu binden, und mit mindestens einem Sensor (11) zur Erfassung von Messsignalen für die Anzahl oder die Dichte der Bindungsereignisse an den einzelnen Teststellen (7), **dadurch gekennzeichnet, dass** bei den einzelnen Teststellen (7) der Abstand zwischen der Teststelle (7) und einem dieser jeweils in Richtung der Normalen auf die Erstreckungsebene der Teststelle gegenüberliegenden Wandungsbereich der Messkammer (3) unterschiedlich groß ist.

7. Vorrichtung (1) nach Anspruch 6, **dadurch gekennzeichnet, dass** der mindestens eine Sensor mit einer Auswerteeinrichtung (1 3) verbunden ist, dass die Auswerteeinrichtung (13) einen Referenzwertgeber (14) zum Bereitstellen mindestens eines Referenzwerts oder Referenzbereichs für die Messsignale der Teststellen (7) aufweist, dass die Auswerteeinrichtung (13) mindestens eine Vergleichseinrichtung (12) zum Vergleichen der Messsignale der einzelnen Teststellen (7) mit dem Referenzwert oder Referenzwertbereich hat, und dass die Vergleichseinrichtung (12) derart mit einer Schalteinrichtung (17) verbunden ist, dass das Messsignal, welches den geringsten Abstand zum Referenzwert oder Referenzwertbereich aufweist oder mit diesem übereinstimmt, an einen Messsignalausgang (18) der Schalteinrichtung (17) anlegbar ist.

8. Vorrichtung (1) nach Anspruch 6 oder 7, **dadurch gekennzeichnet, dass** der Referenzwert dem Wendepunkt (15) einer sigmoidalen Kalibrationskurve (16) entspricht, die unterschiedlichen Werten für die Ligandenkonzentration jeweils einen Messwert für die Teststelle(en) (7) zuordnet.

9. Vorrichtung (1) nach einem der Ansprüche 6 bis 8, **dadurch gekennzeichnet, dass** mindestens eine Teststelle (7) an einer ersten Kammerwand und der dieser Teststelle (7) gegenüberliegende Wandungsbereich an einer zweiten Kammerwand angeordnet sind, und dass diese Kammerwände unter einem Winkel (α) relativ zueinander geneigt sind.

10. Vorrichtung (1) nach einem der Ansprüche 6 bis 9, **dadurch gekennzeichnet, dass** der Winkel (α) mindestens 2,5°, insbesondere mindestens 5° und vorzugsweise mindestens 10° beträgt.

11. Vorrichtung (1) nach einem der Ansprüche 6 bis 10, **dadurch gekennzeichnet, dass** zwischen mindestens zwei Wandungsbereichen eine Stufe (19) oder ein Absatz gebildet ist, und dass jedem dieser Wandungsbereiche jeweils mindestens eine Teststelle (7) zugeordnet ist.

12. Vorrichtung (1) nach einem der Ansprüche 6 bis 11, **dadurch gekennzeichnet, dass** die erste Kammerwand und/oder die dieser gegenüberliegende zweite Kammerwand in quer zueinander verlaufenden Richtungen jeweils mehrere nebeneinander angeordnete Wandungsbereiche aufweisen, zwischen denen Stufen (19) oder Absätze gebildet sind.

13. Vorrichtung (1) nach einem der Ansprüche 6 bis 12, **dadurch gekennzeichnet, dass** die Wandungsbereiche, zwischen denen die Stufen (19) oder Absätze gebildet sind, als Vielecke ausgestaltet und vorzugsweise schachbrettmusterartig oder wabenartig angeordnet sind.

14. Vorrichtung (1) nach einem der Ansprüche 6 bis 1 3, **dadurch gekennzeichnet, dass** mindestens eine erste Teststelle (7) in Richtung der Normalen auf die von ihr aufgespannte Ebene einen ersten Abstand zum gegenüberliegenden Wandungsbereich und wenigstens eine zweite Teststelle (7) in Richtung der Normalen auf die von ihr aufgespannte Ebene einen zweiten Abstand zum gegenüberliegenden Wandungsbereich aufweist, und dass der erste Abstand mindestens das 1,5-fache, insbesondere mindestens das zweifache und vorzugsweise mindestens das vierfache des zweiten Abstands beträgt.

15. Vorrichtung (1) nach einem der Ansprüche 6 bis 14, **dadurch gekennzeichnet, dass** an den einzelnen Teststellen (7) jeweils mindestens ein Sensor (11) zum Detektieren der Liganden-Rezeptor-Komplexe in der Wandung der Messkammer (3) angeordnet ist, und dass der wenigstens eine Rezeptor (8) auf dem Sensor (11) immobilisiert ist.

16. Vorrichtung (1) nach einem der Ansprüche 6 bis 14, **dadurch gekennzeichnet, dass** mindestens ein Sensor (11) ein ionenselektiver Feldeffekttransistor ist.

17. Vorrichtung (1) nach einem der Ansprüche 6 bis 16, **dadurch gekennzeichnet, dass** mindestens ein Sensor (11) ein optischer Strahlungsempfünger ist, der für eine in Abhängigkeit von der Bindung des Liganden (2) an den Rezeptor (8) anregbare Lumineszenzstrahlung empfindlich ist.

## Claims

1. Method for measuring the concentration of at least one ligand (2) contained in a sample to be examined, in which at least one receptor (8) is in each case immobilized at at least two test sites (7) which are arranged on the surface of at least one substrate, which receptor is suited to specifically bind to the ligand (2) when contacting the latter, the sample being brought into contact with the test sites (7) for a predetermined length of time such that diffusion volumes with different sizes adjoin the receptors (8) which are immobilized at the test sites (7) in the sample, from which diffusion volumes the at least one ligand (2) can in each case diffuse to the relevant receptor (8) during the predetermined length of time if the ligand (2) is contained in the diffusion volume, respectively one measurement signal for the number or the frequency of binding events at the test site (7) being detected for each test site (7) after the predetermined length of time has expired.

2. Method according to Claim 1, **characterized in that** the measurement values are in each case compared to a predetermined reference value or reference range and **in that** the concentration of the ligand (2) in the sample is determined with the aid of the measurement signal which has the smallest distance from the reference value or reference value range or which corresponds thereto.

3. Method according to Claim 1 or 2, **characterized in that** the reference value range lies between 25% and 75%, in particular between 40% and 60%, of the measurement signal measured at a test site (7) if substantially all receptors (8) of the test site (7) are bound to a ligand (2), and **in that** the reference value preferably corresponds to the turning point (15) of a sigmoid calibration curve (16), which respectively assigns one measurement value for the test site (7) to different values of the ligand concentration.

4. Method according to one of Claims 1 to 3, **characterized in that** the diffusion volumes with different sizes are formed by at least one delimiting wall which adjoins the sample at a distance from the test site.

5. Method according to one of Claims 1 to 4, **characterized in that** the diffusion volumes with different sizes are formed by the sample being arranged with a different filling height at the individual test sites (7).

6. Apparatus (1) for measuring the concentration of at least one ligand contained in a sample to be examined, with a measurement chamber (3) with at least one inlet opening (4) for the sample, which measurement chamber has an inner cavity (6) with delimiting walls, with at least two test sites (7) being arranged on at least one delimiting wall, in each case at least one receptor (8) being immobilized at the test sites, which receptor is suited to specifically bind to the ligand (2) when contacting the latter, and with at least one sensor (11) for detecting measurement signals for the number or the frequency of the binding events at the individual test sites (7), **characterized in that**, at the individual test sites (7), there are different distances between the test site (7) and a wall region of the measurement chamber (3) respectively lying opposite said test site in the direction of the normal of the plane of extent of the test site.

7. Apparatus (1) according to Claim 6, **characterized in that** the at least one sensor is connected to an evaluation device (13), **in that** the evaluation device (13) has a reference value provider (14) for providing at least one reference value or reference range for the measurement signals of the test sites (7), **in that** the evaluation device (13) has at least one comparator device (12) for comparing the measurement signals of the individual test sites (7) with the reference value or the reference value range, and **in that** the comparator device (12) is connected to a switching device (17) such that the measurement signal, which has the smallest distance from the reference value or reference value range or which corresponds thereto, can be applied to a measurement signal output (18) of the switching device (17).

8. Apparatus (1) according to Claim 6 or 7, **characterized in that** the reference value corresponds to the turning point (15) of a sigmoid calibration curve (16), which respectively assigns one measurement value for the test site(s) (7) to different values of the ligand concentration.

9. Apparatus (1) according to one of Claims 6 to 8, **characterized in that** at least one test site (7) is arranged on a first chamber wall and the wall region lying opposite this test site (7) is arranged on a second chamber wall, and **in that** these chamber walls are inclined with respect to one another at an angle (α).

10. Apparatus (1) according to one of Claims 6 to 9, **characterized in that** the angle (α) is at least 2.5°, in particular at least 5° and preferably at least 10°.

11. Apparatus (1) according to one of Claims 6 to 10, **characterized in that** a step (19) or a ledge is formed between at least two wall regions and **in that** each of these wall regions respectively has at least one test site (7) assigned to it.

12. Apparatus (1) according to one of Claims 6 to 11, **characterized in that** the first chamber wall and/or the second chamber wall lying opposite the former respectively have a number of wall regions, which are arranged next to one another in directions which run transversely with respect to one another, between which steps (19) or ledges are formed.

13. Apparatus (1) according to one of Claims 6 to 12, **characterized in that** the wall regions, between which the steps (19) or ledges are formed, are designed as polygons and are preferably arranged in a chequer-board pattern or a honeycomb pattern.

14. Apparatus (1) according to one of Claims 6 to 13, **characterized in that** at least one first test site (7) has, in the direction of the normal of the plane spanned by said test site, a first spacing from the oppositely lying wall region and at least one second test site (7) has, in the direction of the normal of the plane spanned by said test site, a second spacing from the oppositely lying wall region, and **in that** the first spacing is at least 1.5 times, in particular at least twice and preferably at least four times the second spacing.

15. Apparatus (1) according to one of Claims 6 to 14, **characterized in that** at least one sensor (11) for detecting the ligand-receptor complexes is respectively arranged in the wall of the measurement chamber (3) at the individual test sites (7) and **in that** the at least one receptor (8) is immobilized on the sensor (11).

16. Apparatus (1) according to one of Claims 6 to 14, **characterized in that** at least one sensor (11) is an ion-selective field effect transistor.

17. Apparatus (1) according to one of Claims 6 to 16, **characterized in that** at least one sensor (11) is an optical radiation receiver which is sensitive to luminescent radiation which can be excited as a function of the binding of the ligand (2) to the receptor (8).

## Revendications

1. Procédé de mesure de la concentration d'au moins un ligand (2) contenu dans un échantillon à analyser, dans lequel, à au moins deux points de test (7) qui sont disposés à la surface d'au moins un support, est immobilisé respectivement au moins un récepteur (8) qui, en cas de contact avec le ligand (2), est apte à se lier spécifiquement à celui-ci, l'échantillon étant amené en contact pendant une durée prédéterminée avec les points de test (7) de manière telle que, dans l'échantillon, des volumes de diffusion d'importance différente jouxtent les récepteurs (8) immobilisés aux points de test (7), volumes depuis lesquels l'au moins un ligand (2) peut se diffuser pendant la durée prédéterminée respectivement vers le récepteur correspondant (8) lorsque le ligand (2) est contenu dans le volume de diffusion, respectivement un signal de mesure pour le nombre ou la densité des événements de liaison étant enregistré au point de test (7) après écoulement de la durée prédéterminée pour chaque point de test (7).

2. Procédé selon la revendication 1, **caractérisé en ce que** les valeurs de mesure sont respectivement comparées à une valeur de référence ou plage de valeurs de référence prédéfinie et que, à l'aide du signal de mesure qui présente l'écart le plus réduit par rapport à la valeur de référence ou à la plage de valeurs de référence ou coïncide avec celle-ci, la concentration du ligand (2) dans l'échantillon est définie.

3. Procédé selon la revendication 1 ou 2, **caractérisé en ce que** la plage de valeurs de référence est comprise entre 25 % et 75 %, notamment 40 % et 60 %, du signal de mesure qui est mesuré à un point de test (7) lorsque sensiblement tous les récepteurs (8) du point de test (7) sont liés à un ligand (2) et que la valeur de référence équivaut de préférence au point d'inversion (15) d'une courbe de calibrage sigmoïde (16) qui associe respectivement une valeur de mesure pour le point de test (7) à des valeurs différentes de concentration de ligand.

4. Procédé selon l'une quelconque des revendications 1 à 3, **caractérisé en ce que** les volumes de diffusion d'importance différente sont constitués par au moins une paroi de limitation qui jouxte à distance le point de test.

5. Procédé selon l'une quelconque des revendications 1 à 4, **caractérisé en ce que** les volumes de diffusion d'importance différente sont constitués du fait que l'échantillon est disposé avec un niveau de remplissage différent aux points de test individuels (7).

6. Dispositif (1) pour la mesure de la concentration d'au moins un ligand contenu dans un échantillon à analyser, comportant au moins une ouverture d'entrée (4) pour la chambre de mesure (3) contenant l'échantillon, laquelle chambre de mesure comporte une cavité intérieure (6) avec des parois de limitation, sur au moins une paroi de limitation étant disposés au moins deux points de test (7) au niveau desquels respectivement est immobilisé au moins un récepteur (8) qui est apte, en cas de contact avec le ligand (2), à se lier spécifiquement à celui-ci, et comportant au moins un capteur (11) pour enregistrer des signaux de mesure pour le nombre et la densité des événements de liaison aux points de test individuels (7), **caractérisé en ce que**, aux points de test individuels (7), la distance entre le point de test (7) et une zone de paroi, opposée respectivement à celui-ci en direction de la perpendiculaire sur le plan d'extension du point de test, de la chambre de mesure (3) est de longueur différente.

7. Dispositif (1) selon la revendication 6, **caractérisé en ce que** l'au moins un capteur est raccordé à un dispositif d'exploitation (13), que le dispositif d'exploitation (13) comporte un indicateur de valeurs de référence (14) destiné à fournir au moins une valeur de référence ou une plage de valeurs de référence pour les signaux de mesure des points de test (7), que le dispositif d'exploitation (13) comporte au moins un dispositif comparatif (12) pour comparer les signaux de mesure des points de test individuels (7) à la valeur de référence ou plage de valeurs de référence et que le dispositif comparatif (12) est raccordé à un dispositif de manoeuvre (17) d'une manière telle que le signal de mesure présentant le plus faible écart par rapport à la valeur de référence ou plage de valeurs de référence ou coïncide avec celle-ci peut être appliqué à une sortie de signal de mesure (18) du dispositif de manoeuvre (17).

8. Dispositif (1) selon la revendication 6 ou 7, **caractérisé en ce que** la valeur de référence correspond au point d'inversion (15) d'une courbe de calibrage sigmoïde (16) qui associe respectivement à des valeurs différentes pour la concentration de ligand une valeur de mesure pour le(s) point(s) de test (7).

9. Dispositif (1) selon l'une quelconque des revendications 6 à 8, **caractérisé en ce qu'**au moins un point de test (7) est disposé au niveau d'une première paroi de chambre et que la zone de paroi opposée à ce point de test (7) est disposée au niveau d'une deuxième paroi de chambre et que ces parois de chambre sont inclinées l'une par rapport à l'autre d'un angle (α).

10. Dispositif (1) selon l'une quelconque des revendications 6 à 9, **caractérisé en ce que** l'angle (α) s'élève à au moins 2,5°, notamment au moins 5° et de préférence au moins 10°.

11. Dispositif (1) selon l'une quelconque des revendications 6 à 10, **caractérisé en ce que**, entre au moins deux zones de paroi, il est formé un échelon (19) ou un talon et qu'à chacune de ces zones de paroi est associé respectivement au moins un point de test (7).

12. Dispositif (1) selon l'une quelconque des revendications 6 à 11, **caractérisé en ce que** la première paroi de chambre et/ou la deuxième paroi de chambre opposée à celle-ci présentent respectivement, dans des sens s'étendant tranversalement l'un par rapport à l'autre, plusieurs zones de paroi juxtaposées entre lesquelles des échelons (19) ou talons sont formés.

13. Dispositif (1) selon l'une quelconque des revendications 6 à 12, **caractérisé en ce que** les zones de paroi entre lesquelles sont formés les échelons (19) ou talons sont réalisées sous forme de polygones et sont disposées de préférence en motif d'échiquier ou de nid d'abeille.

14. Dispositif (1) selon l'une quelconque des revendications 6 à 13, **caractérisé en ce qu'**au moins un premier point de test (7) présente, en direction de la perpendiculaire vers le plan qu'il circonscrit, une première distance par rapport à la zone de paroi opposée et qu'au moins un deuxième point de test (7) présente, en direction de la perpendiculaire vers le plan qu'il circonscrit, une deuxième distance par rapport à la zone de paroi opposée et que la première distance représente au moins 1,5 fois, notamment au moins deux fois et de préférence au moins quatre fois la deuxième distance.

15. Dispositif (1) selon l'une quelconque des revendications 6 à 14, **caractérisé en ce que**, aux points de test individuels (7), est disposé respectivement au moins un capteur (11) pour détecter les complexes ligand-récepteur dans la paroi de la chambre de mesure (3) et que l'au moins un récepteur (8) est immobilisé sur le capteur (11).

16. Dispositif (1) selon l'une quelconque des revendications 6 à 14, **caractérisé en ce qu'**au moins un capteur (11) est un transistor à effet de champ sélectif d'ions.

17. Dispositif (1) selon l'une quelconque des revendications 6 à 16, **caractérisé en ce qu'**au moins un capteur (11) est un récepteur de rayonnement optique qui est sensible à un rayonnement luminescent pouvant être excité en fonction de la liaison du ligand (2) au récepteur (8).
